# EUROPÄISCHE PATENTSCHRIFT

(11) **EP 1 595 571 B1**
(45) Veröffentlichungstag und Bekanntmachung des Hinweises auf die Patenterteilung: **13.02.2008**
(21) Anmeldenummer: 05103790.1
(22) Anmeldetag: 06.05.2005
(51) Int. Cl.: A61N 1/05

(54) **Elektrodenleitung**
Electrode lead
Fil d'électrode

(30) Priorität: 14.05.2004 DE 102004025101; 09.07.2004 DE 102004034336
(43) Veröffentlichungstag der Anmeldung: 16.11.2005
(73) Patentinhaber: BIOTRONIK CRM Patent AG, 6340 Baar (CH)
(72) Erfinder: Geistert, Dr.Wolfgang, 79618 Rheinfelden (DE)
(74) Vertreter: Lindner-Vogt, Karin L.

(56) Entgegenhaltungen:
- US-A- 5 549 109
- US-A- 5 683 445
- US-A- 5 800 495
- US-A1- 2003 109 914
- US-A1- 2003 171 796
- US-B1- 6 363 287
- US-B1- 6 408 213
- US-B1- 6 529 779

## Beschreibung

Die Erfindung betrifft eine Elektrodenleitung für die intraluminale oder intrakardiale Anwendung, die zum Anschluss an ein Elektrostimulationsgerät, wie einen Herzschrittmacher, Defibrillator, Kardioverter oder dergleichen, geeignet ist. Die Elektrodenleitung besitzt einen längsgestreckten, biegeweichen Elektrodenleitungskörper, welcher ein proximales Ende besitzt, an welchem ein elektrischer Anschluss zum Herstellen einer elektrischen Verbindung mit einem Stimulationsgerät angeordnet ist. Außerdem besitzt der Elektrodenleitungskörper ein distales Ende, an dem wenigstens eine distale Elektrode angeordnet ist, die mit dem elektrischen Anschluss elektrisch verbunden ist und elektrisch leitende Außenoberfläche aufweist. In dem Elektrodenleitungskörper ist ein Lumen zur Aufnahme eines Führungsdrahtes vorgesehen. Dieses Lumen erstreckt sich von einer distalen Öffnung im Bereich des distalen Endes des Elektrodenleitungskörpers bis zu einer proximalen Öffnung, welche proximal der distalen Öffnung, vorzugsweise im Bereich des proximalen Endes des Elektrodenkörpers, angeordnet ist. Die distale Öffnung ist so ausgebildet und angeordnet, dass ein Führungsdraht durch die distale Öffnung aus dem Lumen austreten kann.

Derartige Elektrodenleitungen sind aus dem Stand der Technik bereits bekannt, beispielsweise aus dem US-Patent 5,800,495. Dort ist eine Elektrodenleitung beschrieben, die ein Lumen und eine distale Öffnung aufweist, die zur Aufnahme eines Stiletts geeignet ist.

Dokument US 2003/0171796 offenbart eine Elektrodenleitung gemäß dem Oberbegriff des Anspruchs 1.

Ausgehend von diesem Stand der Technik soll eine Elektrodenleitung geschaffen werden, die dem Arzt weitere Anwendungsbereiche erschließt.

Erfindungsgemäß wird dieses Ziel durch eine Elektrodenleitung der eingangs genannten Art erreicht, bei der die distale Elektrode der Elektrodenleitung und die distale Öffnung, die den Austritt des Führungsdrahtes im Bereich des distalen Endes der Elektrodenleitung erlaubt, mit Bezug auf die Längsrichtung des Elektrodenleitungskörpers seitlich zueinander versetzt angeordnet sind. Dieser seitliche Versatz erlaubt es, eine sichere Wandständigkeit der distalen Elektrode zu erreichen, wenn die Elektrodenleitung über einen Führungsdraht in ein Blutgefäß eingeführt wird. Um bei einer derartigen, gegenüber der distalen Öffnung seitlich versetzten Anordnung der distalen Elektrode ein Verhaken des distalen Endes des Elektrodenleitungskörpers während des Einführens der Elektrodenleitung entlang einem Führungsdraht zu verhindern, ist außerdem vorgesehen, dass die distale Öffnung wenigstens neben einem proximalen Ende der distalen Elektrode oder vor diesem - also näher am distalen Ende des Elektrodenleitungskörpers, als das proximale Ende der distalen Elektrode - angeordnet ist.

Der Elektrodenleitungskörper ist vorzugsweise wenigstens im Bereich seines distalen Endes derart vorgebogen, dass er sich in Richtung der lateral angeordneten distalen Elektrode krümmt. Dies sichert eine gute Wandständigkeit der lateral angeordneten Elektrode. Seine Krümmung nimmt der Elektrodenleitungskörper wenigstens nach Entfernen eines Führungsdrahtes an, d.h. der Elektrodenleitungskörper kann so gestaltet sein, dass seine Vorkrümmung durch einen eingeführten Führungsdraht oder ein Stillet aufgehoben wird.

Die Vorkrümmung verläuft vorzugsweise wenigstens in einer durch die Längsrichtung des Elektrodenleitungskörpers sowie die distale Öffnung und die distale Elektrode definierten Ebene in Richtung der distalen Elektrode.

In einer besonders bevorzugten Variante der Erfindung befindet sich die distale Öffnung in einer distalen Stirnseite des Elektrodenleitungskörpers, und ist bezüglich einer Mittellinie des Elektrodenleitungskörpers nicht zentral, sondern seitlich versetzt angeordnet.

Außerdem ist auf dem Elektrodenleitungskörper neben der distalen Elektrode vorzugsweise wenigstens eine zweite Elektrode vorgesehen, die bevorzugt die Form einer Ringelektrode hat, welche proximal der distalen Elektrode angeordnet ist und einen Längsabstand zu dieser besitzt sowie das Lumen im Inneren des Elektrodenleitungskörpers umschließt. Diese bevorzugte Ausführungsvariante erlaubt eine bipolare Stimulation beziehungsweise das bipolare Erfassen von Herzpotentialen (bipolares Sensing). Anstelle einer einzigen distalen Elektrode können auch zwei distale Elektroden vorgesehen sein, die beide jeweils seitlich bezüglich der distalen Öffnung des Lumens versetzt angeordnet sind. Insbesondere bei der letztgenannten Ausführungsvariante befindet sich die distale Öffnung des Lumens vorzugsweise in einer distalen Stirnfläche des Elektrodenleitungskörpers und kann in diesem Fall auch zentral angeordnet sein, da die beiden distalen Elektroden zu beiden Seiten der distalen Öffnung lateral (seitlich) versetzt angeordnet sein können.

Eine alternative, ebenfalls bevorzugte Ausführungsvariante der Elektrodenleitung mit einer distalen Öffnung in einem Längsabstand zu einem proximalen Ende der distalen Elektrode, besitzt in ihrem distal der distalen Öffnung befindlichen Längsabschnitt einen geringeren Durchmesser als der Elektrodenleitungskörper in seinem proximal an die distale Öffnung anschließenden Längsabschnitt besitzt. Bei dieser Variante ist die distale Öffnung haifischmaulartig neben der distalen Elektroden angeordnet, wobei die distale Elektrode sich proximal über die distale Öffnung hinaus erstreckt.

Eine bevorzugte Ausführungsvariante besteht in einem System mit einer Elektrodenleitung der zuvor beschriebenen Art und mit einem Führungsdraht (14). Der Führungsdraht ist in diesem Fall entlang des größten Teils seiner Länge nach außen elektrisch isoliert und besitzt eine Elektrodenoberfläche an seinem distalen Ende, die elektrisch mit einem proximalen Ende des Führungsdrahtes verbunden ist und die als Stimulations- und/oder Abfühlelektrode ausgebildet ist. Die Idee besteht darin, anstelle eines "normalen" Führungsdrahtes einen weitestgehend isolierten Führungsdraht im Zusammenhang mit der hier beschriebenen Elektrodenleitung zu verwenden. Bei einem solchen Führungsdraht ist nur ein kleiner Abschnitt am oder in der Nähe des distalen Endes und ein kleiner Abschnitt am oder in der Nähe des proximalen Endes nicht isoliert. Der kleine Abschnitt in der Nähe des distalen Endes kann als aktive Elektrode gebraucht werden, um an der potenziellen Positionierungsstelle der Elektrode Herzpotenziale abzunehmen oder das Herz zu stimulieren. Der kleine Abschnitt in der Nähe des proximalen Endes dient dann als Anschlussstelle für ein Kabel zu einer Wahrnehmungs- und/oder Stimulationsvorrichtung. Die beiden nicht isolierten Abschnitte sind elektrisch miteinander verbunden. Das Ganze dient dazu, den optimalen Platzierungsort für die Elektrode zu sondieren.

Eine alternative Ausführungsvariante besteht in einem System mit einer Elektrodenleitung der zuvor beschriebenen Art als erste Elektrodenleitung in deren Lumen eine zweite, dünnere, relativ zur ersten Elektrodenleitung längsverschiebliche Elektrodenleitung eingeführt ist. Die zweite Elektrodenleitung trägt wenigstens eine Ring- oder Spitzenelektrode an ihrem distalen Ende oder in dessen Nähe. Die Ring- oder Spitzenelektrode ist als Stimulations- und/oder Abfühlelektrode ausgebildet und mit einem proximalen Ende der zweiten Elektrodenleitung elektrisch verbunden. Die zweite Elektrodenleitung unterscheidet sich von dem zuvor beschriebenen Führungsdraht dadurch, dass ihre Ring- oder Spitzenelektrode mit einem Elektrodenleitungsstecker am proximalen Ende der zweiten Elektrodenleitung elektrisch verbunden ist, der es erlaubt, die zweite Elektrodenleitung an ein elektrisch aktives Medizinisches Gerät, insbesondere an ein Implantat wie einen Herzschrittmacher, Defibrillator oder dergleichen anzuschließen.

Mit der sehr dünnen, zweiten Elektrodenleitung können "tiefer" im verzweigten Gefäßsystem liegende Positionen erreicht werden. Auf diese Weise können multipolare Elektrodensysteme realisiert werden. Beispielsweise kann zwischen einem Pol (Spitzen- oder Ringelektrode) der sehr dünnen, zweiten Elektrodenleitung und einem Pol (Elektrode) der ersten Elektrodenleitung stimuliert oder Herzpotenziale abgegriffen werden.

In einer vorteilhaften Ausführungsform aller hier beschriebenen (ersten) Elektrodenleitungen ist in der Nähe der Austrittsöffnung für den Führungsdraht oder die zweite Elektrodenleitung eine Dichtung untergebracht, die verhindert, dass Blut in das Führungsdrahtlumen bzw. die Elektrodenleitung eindringt

Die Erfindung soll nun anhand von Ausführungsbeispielen mit Hilfe der Figuren näher erläutert werden. Von den Figuren zeigen:
- Figur 1:: eine Variante, bei der die distale Öffnung neben dem proximalen Ende einer distalen Elektrode angeordnet ist;
- Figur 2:: eine Variante ähnlich Figur 1, bei der sich die distale Elektrode auch proximal der distalen Öffnung erstreckt;
- Figur 3.: eine Variante mit zentraler distaler Öffnung und lateral angeordneter distaler Elektrode;
- Figur 4:: eine Variante ähnlich Figur 3 mit zwei lateral angeordneten distalen Elektroden;
- Figur 5:: eine Variante ähnlich Figur 1 mit einem Elektrodenleitungskörper, der wenigstens im Bereich des dargestellten distalen Endes vorgekrümmt ist;
- Figur 6:: Ein System, umfassend eine erste Elektrodenleitung entsprechend den Figuren 1 bis 5 und eine dünnere, in das Lumen der ersten Elektrodenleitung eingeführte zweite Elektrodenleitung; und
- Figur 7:: eine schematische Schnittdarstellung des distalen Endes einer Elektrodenleitung entsprechend den Figuren 1 bis 5 zur Darstellung einer Lippendichtung in der Nähe des distalen Endes des Lumens.

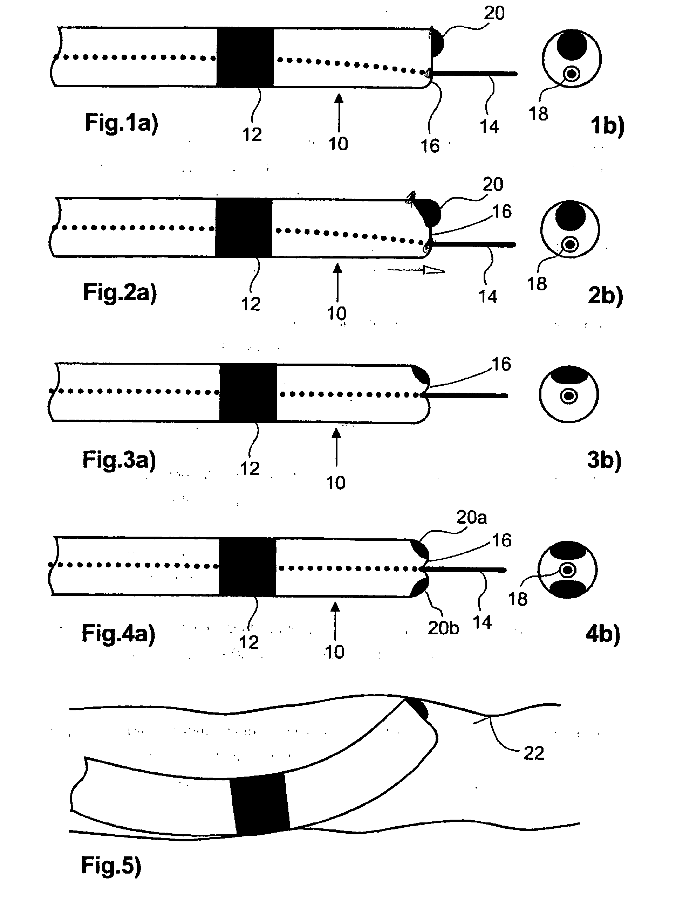

Die Figuren 1 bis 5 zeigen jeweils das distale Ende einer Elektrodenleitung 10. Diese Elektrodenleitung 10 besitzt die üblichen, nicht dargestellten Merkmale einer Elektrodenleitung, nämlich ein proximales Ende mit einem elektrischen Anschluss zum Anschluss der Elektrodenleitung an ein Elektrostimulationsgerät, beispielsweise einen implantierbaren Herzschrittmacher oder einen implantierbaren Kardioverter/Defibrillator. Der Elektrodenleitungskörper der Elektrode 10, dessen distaler Endabschnitt in den Figuren 1 bis 4 dargestellt ist, wird üblicherweise von einem helixartig gewendelten Leiter gebildet, der der Elektrodenleitung die gewünschte Biegeweichheit sowie Festigkeit verleiht und im übrigen wenigstens eine Elektrode, beispielsweise eine Ringelektrode 12 mit dem elektrischen Anschluss am proximalen Ende der Elektrodenleitung elektrisch verbindet. Die in den Figuren nicht dargestellte Helixwendel ist auf der Außenseite üblicherweise von einer elastischen Hülle, beispielsweise aus Silikon umgeben. Die Helixwendel schließt im Inneren ein Lumen ein, das der Führung eines Führungsdrahtes 14 dient, der in den Figuren 1 bis 4 der besseren Erläuterung halber dargestellt ist, obwohl er nicht Teil der Erfindung ist.

Das sich im Inneren des Elektrodenleitungskörpers erstreckende Lumen, welches nicht näher dargestellt ist, aber durch den gestrichelt dargestellten Führungsdraht angedeutet ist, endet im Bereich eines distalen Endes 16 der Elektrodenleitung 10 in einer distalen Öffnung 18. Durch die distale Öffnung 18 kann der Führungsdraht 14 aus dem Lumen innerhalb des Elektrodenleitungskörpers heraustreten.

Im hier relevanten Zusammenhang von Bedeutung ist weiterhin jeweils eine distale Elektrode 20. In Figur 4 sind zwei distale Elektroden 20a und 20b vorgesehen.

In den Figuren 1 bis 4 ist jeweils eine Seitenansicht des distalen Endes der Elektrodenleitung 10 dargestellt (Figuren 1a bis 4a) sowie eine Ansicht auf die distale Stirnseite einer jeweiligen Elektrodenleitung 10 (Figuren 1b bis 4b).

Allen Figuren zu entnehmen ist, dass die jeweilige distale Elektrode 20, beziehungsweise die distalen Elektroden 20a und 20b bezüglich der jeweiligen distalen Öffnung 18 lateral versetzt angeordnet sind. Der laterale Versatz bezieht sich auf die Längsrichtung der Elektrodenleitung 10 und ist insbesondere in den Stirnansichten in den Figuren 1b bis 4b deutlich zu erkennen. Wie insbesondere die Stirnansichten in den Figuren 1b bis 4b zeigen, sind die distalen Elektroden 20 beziehungsweise 20a und 20b jeweils neben der distalen Öffnung 18 angeordnet. Die distalen Elektroden 20 beziehungsweise 20a und 20b haben somit in vorteilhafter Weise selbst keine Öffnung, durch die der Führungsdraht hindurchtreten muss.

Auf diese Weise können die Ringelektroden 12 und die distalen Elektroden (Spitzenelektroden oder Tip-Elektroden) 20 beziehungsweise 20a und 20b wie herkömmliche Ring- und Spitzenelektroden ausgebildet sein und beispielsweise in einer bevorzugten Ausführungsvariante fraktal mittels Iridium oder Titannitrit oder Iridiumoxid beschichtet sein.

Figur 5 zeigt eine Elektrodenleitung entsprechend Figur 1, deren distaler Endabschnitt derart vorgebogen ist, dass sich der Elektrodenleitungskörper nach Entfernen des Führungsdrahtes krümmt. Auf diese Weise lässt sich eine sichere Wandständigkeit der lateral angeordneten distalen Elektrode 20 erzielen, wie der Figur 5 zu entnehmen ist, wenn man die relative Lage der distalen Elektrode 20 zu einer der Illustration halber angedeuteten Gefäßwand 22 betrachtet.

Vorzugsweise ist die Krümmung dergestalt, dass nicht nur dir distale Elektrode 20 sondern auch die proximale Elektrode 12 gegen die Gefäßwand gedrückt wird.

Vorgeformte Elektrodenleitungen, die sich nach Entfernen eines Führungsdrahtes oder eines Stiletts aufgrund ihrer Vorspannung krümmen, sind grundsätzlich bekannt. Die Vorformung kann unter anderem durch eine entsprechend geformte Helixwendel des Elektrodenleitungskörpers oder durch eine entsprechende äußere Kunststoffhülle erzielt werden. Im Zusammenhang mit den hier dargestellten Ausführungsvarianten mit lateral angeordneter distaler Elektrode kommt der Vorformung jedoch jeweils eine besondere Bedeutung zu.

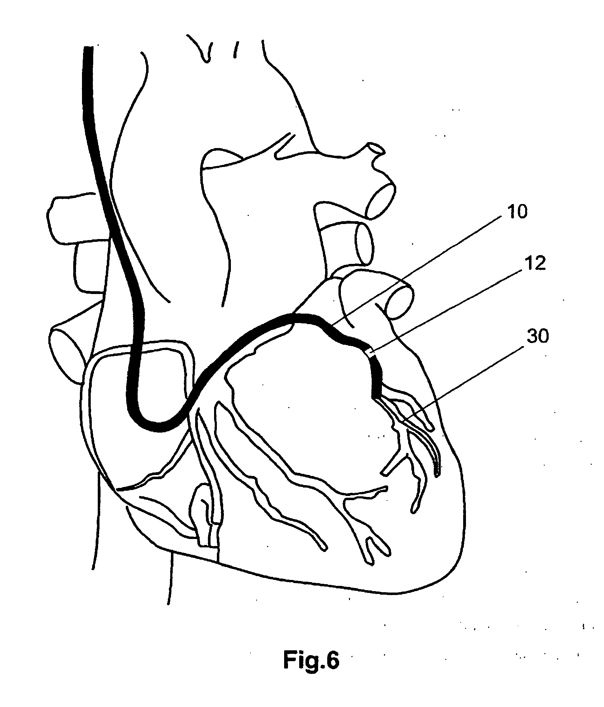

Figur 6 ist eine schematische Darstellung einer Elektrodenleitung 10, in deren Lumen eine zweite, dünnere Elektrodenleitung 30 eingeführt ist. Wie der Figur 6 zu entnehmen ist, tritt die zweite Elektrodenleitung 30 am distalen Ende der ersten Elektrodenleitung 10 aus dieser heraus und ragt von dort ausgehend in weitere, feiner verästelte Blutgefäße eines in Figur 6 dargestellten Herzens. Die erste Elektrodenleitung 10 ist durch das rechte Atrium des Herzens in den Coronar Sinus eingeführt und von dort aus in einer von Coronar Sinus abzweigende Lateralvene, in der schließlich die distale Elektrode 20 und ggf. die proximale Elektrode 12 positioniert sind. Auch die zweite Elektrodenleitung 30 trägt eine in Figur 6 nicht erkennbare Spitzenelektrode an ihrem distalen Ende 31.

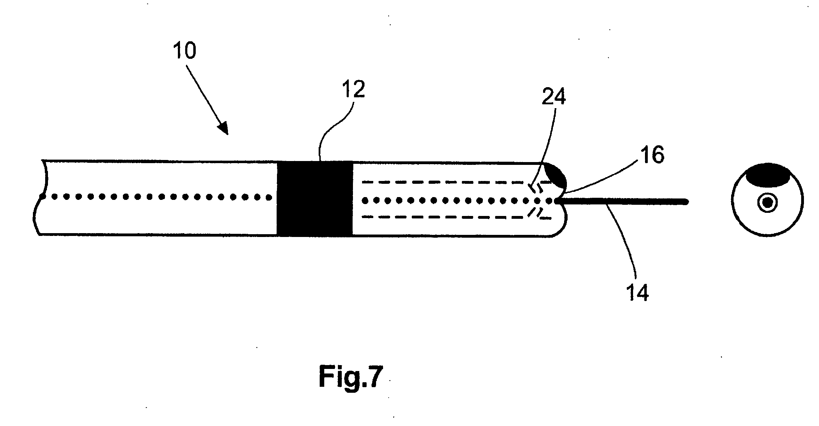

Figur 7 zeigt schließlich in schematischer Darstellung das distale Ende einer ersten Elektrodenleitung 10, ähnlich Figur 3. Die Darstellung zeigt schematisch, wie im Lumen der Elektrodenleitung 10 in der Nähe des distalen Endes 16 eine Lippendichtung 24 angeordnet ist, durch die der Führungsdraht 14 hindurchtreten kann. Diese Lichtlippendichtung 24 verhindert ein Eindringen von Blut in das Lumen der Elektrodenleitung 10, und zwar sowohl in dem Fall, in dem ein Führungsdraht 14 durch die Lippendichtung 24 hindurchtritt, wie in Figur 7 dargestellt, als auch in dem Fall, in dem kein Führungsdraht in das Lumen der Elektrodenleitung 10 eingeführt ist. Im letztgenannten Fall berühren sich die Lippen der Lippendichtung und verschließen das distale Ende des Lumens der Elektrodenleitung 10.

## Patentansprüche

1. Elektrodenleitung für die intraluminale oder intrakardiale Anwendung zum Anschluss an einen implantierbaren Herzschrittmacher, einen implantierbaren Defibrillator oder einen implantierbaren Kardioverter,
mit einem längsgestreckten, biegeweichen Elektrodenleitungskörper (10), welcher ein proximales Ende besitzt, an welchen ein elektrischer Anschluss zum Herstellen einer elektrischen Verbindung mit einen Elektrostimulationsgerät angeordnet ist,
sowie ein distales Ende (16), an dem wenigstens eine distale Elektrode (20; 20a, 20b) angeordnet ist, die mit dem elektrischen Anschluss elektrisch verbunden ist und eine elektrisch leitende Außenoberfläche aufweist,
und in welchen ein Lumen zur Aufnahme eines Führungsdrahtes (14) oder dergleichen vorgesehen ist, welche sich von einer proximalen Öffnung im Bereich des proximalen Endes des Elektrodenleitungskörpers bis zu einer distalen Öffnung (18) im Bereich des distalen Endes des Elektrodenleitungskörpers (10) erstreckt, wobei die distale Öffnung (18) derart ausgebildet und angeordnet ist, dass ein Führungsdraht durch die distale Öffnung (18) aus dem Lumen austreten kann,
wobei die distale Elektrode (20;20a,20b) selbst keine Öffnung durch die der Führungsdraht hindurchtreten muss aufweist, und die distale Elektrode (20; 20a, 20b) und die distale Öffnung (18) im Bereich des distalen Endes (16) des Elektrodenleitungskörpers (10) bezüglich einer Längsrichtung des Elektrodenleitungskörpers (10) lateral zueinander versetzt angeordnet sind, so dass ein aus der distalen Öffnung (18) austretender Führungsdraht seitlich versetzt an der distalen Elektrode (20; 20a, 20b) vorbeigeführt wird
**dadurch gekennzeichnet, dass**
die distale Öffnung (18) ohne Längsversatz neben einem proximalen Ende der distalen Elektrode oder mit einem Längsversatz distal gegenüber dem proximalen Ende der distalen Elektrode versetzt angeordnet ist.

2. Elektrodenleitung nach Anspruch 1, **dadurch gekennzeichnet, dass** die distale Elektrode (20) als Tip-Elektrode ausgebildet und am distalen Ende des Elektrodenleitungskörpers angeordnet ist.

3. Elektrodenleitung nach Anspruch 2, **dadurch gekennzeichnet, dass** der Elektrodenleitungskörper (19) wenigstens im Bereich seines distalen Endes derart vorgebogen ist, dass sich der Elektrodenleitungskörper wenigstens nach Entfernen eines Führungsdrahtes wenigstens in einer durch die Längsrichtung des Elektrodenleitungskörpers sowie die distale Öffnung und die distale Elektrode definierten Ebene in Richtung der distalen Elektrode krümmt.

4. Elektrodenleitung nach Anspruch 2, **dadurch gekennzeichnet, dass** am distalen Ende des Elektrodenleitungskörper zwei distale Elektroden (20a, 20b) seitlich der distalen Öffnung (18) angeordnet sind.

5. Elektrodenleitung nach Anspruch 4, **dadurch gekennzeichnet, dass** die distale Öffnung (18) zentral zwischen den beiden distalen Elektroden (20a, 20b) angeordnet ist.

6. Elektrodenleitung nach Anspruch 2, **dadurch gekennzeichnet, dass** die distale Öffnung (18) bezüglich eines proximalen Endes der distalen Elektrode in distale Richtung versetzt angeordnet ist.

7. Elektrodenleitung nach Anspruch 6, **dadurch gekennzeichnet, dass** der Elektrodenleitungskörper (10) in einem proximal der distalen Öffnung (18) befindlichen proximalen Längsabschnitt einen größeren Außendurchmesser aufweist als die distale Elektrode (20) in ihrem distal der distalen Öffnung gelegenen distalen Längsabschnitt zwischen der distalen Öffnung (18) und dem distalen Ende des Elektrodenleitungskörpers (10).

8. Elektrodenleitung nach Anspruch 2, **dadurch gekennzeichnet, dass** die distale Öffnung (18) bezüglich Längsrichtung des Elektrodenleitungskörpers (10) ohne Längsversatz relativ zum proximalen Ende der distalen Elektrode (20) angeordnet ist.

9. Elektrodenleitung nach einem der Ansprüche 1 bis 8, **dadurch gekennzeichnet, dass** proximal der distalen Elektrode (20) eine proximale Elektrode (12) angeordnet ist, die einen Abstand zum distalen Ende (16) des Elektrodenleitungskörpers (10) hat.

10. Elektrodenleitung nach Anspruch 9, **dadurch gekennzeichnet, dass** die proximale Elektrode (12) eine Ringelektrode ist, die das Lumen umschließt.

11. Elektrodenleitung nach einem der Ansprüche 1 bis 10 mit einem Führungsdraht (14), **dadurch gekennzeichnet, dass** der Führungsdraht entlang des größten Teils seiner Länge eine außenliegende elektrische Isolierung aufweist und eine Elektrodenoberfläche an seinem distalen Ende oder in dessen Nähe aufweist, die elektrisch mit einem proximalen Ende des Führungsdrahtes verbunden ist und die als Stimulations- und/oder Abfühlelektrode ausgebildet ist.

12. Elektrodenleitung nach einem der Ansprüche 1 bis 10, **gekennzeichnet durch** eine in das Lumen eingeführte, relativ zur ersten Elektrodenleitung längsverschiebliche zweite Elektrodenleitung mit wenigstens einer Ring- oder Spitzenelektrode am distalen Ende der zweiten Elektrodenleitung oder in dessen Nähe, wobei die Ring- oder Spitzenelektrode als Stimulations- und/oder Abfühlelektrode ausgebildet und mit einem proximalen Ende der zweiten Elektrodenleitung elektrisch verbunden ist.

13. Elektrodenleitung nach einem der Ansprüche 1 bis 12, **gekennzeichnet durch** eine Dichtung (24) im Bereich eines distalen Endes des Lumens, die ausgebildet ist, das Hindurchführen eines Führungsdrahtes oder dergleichen zu erlauben und das Lumen gegen ein Eindringen von Blut abzudichten.

14. Elektrodenleitung nach Anspruch 13, **dadurch gekennzeichnet, dass** die Dichtung als Lippendichtung (24) ausgebildet ist.

## Claims

1. An electrode lead for intraluminal or intracardial application for connection to an implantable cardiac pacemaker, an implantable defibrillator, or an implantable cardioverter,
having an elongate, flexible electrode lead body (10), which has a proximal end, on which an electrical terminal for producing an electrical connection to an electrode stimulation device is situated,
and a distal end (16), on which at least one distal electrode (20; 20a, 20b) is situated, which is electrically connected to the electrical terminal and has an electrically conductive external surface,
and in which a lumen is provided for receiving a guide wire (14) or the like, which extends from a proximal opening in the area of the proximal end of the electrode lead body up to a distal opening (18) in the area of the distal end of the electrode lead body (10), the distal opening (18) being implemented and situated in such a way that a guide wire may exit from the lumen through the distal opening (18),
the distal electrode (20; 20a, 20b) itself not having an opening through which the guide wire must exit, and the distal electrode (20; 20a, 20b) and the distal opening (18) in the area of the distal end (16) of the electrode lead body (10) being situated laterally offset to one another in relation to longitudinal direction of the electrode lead body (10), so that a guide wire exiting from the distal opening (18) is guided laterally offset past the distal electrode (20; 20a, 20b),
**characterized in that** the distal opening (18) is situated without longitudinal offset adjacent to a proximal end of the distal electrode or with a longitudinal offset distally in relation to the proximal end of the distal electrode.

2. The electrode lead according to Claim 1, **characterized in that** the distal electrode (20) is implemented as a tip electrode and is situated on the distal end of the electrode lead body.

3. The electrode lead according to Claim 2, **characterized in that** the electrode lead body (19) is pre-curved at least in the area of its distal end in such a way that the electrode lead body curves in the direction of the distal electrode at least after removal of a guide wire at least in a plane defined by the longitudinal direction of the electrode lead body as well as by the distal opening and the distal electrode.

4. The electrode lead according to Claim 2, **characterized in that** two distal electrodes (20a, 20b) are situated laterally from the distal opening (18) on the distal end of the electrode lead body.

5. The electrode lead according to Claim 4, **characterized in that** the distal opening (18) is situated centrally between the two distal electrodes (20a, 20b).

6. The electrode lead according to Claim 2, **characterized in that** the distal opening (18) is situated offset in the distal direction in relation to a proximal end of the distal electrode.

7. The electrode lead according to Claim 6, **characterized in that** the electrode lead body (10) has a greater external diameter in a proximal longitudinal section located proximally to the distal opening (18) than the distal electrode (20) in its distal longitudinal section placed distally to the distal opening between the distal opening (18) and the distal end of the electrode lead body (10).

8. The electrode lead according to Claim 2, **characterized in that** the distal opening (18) is situated without longitudinal offset in relation to the proximal end of the distal electrode (20) in regard to the longitudinal direction of the electrode lead body (10).

9. The electrode lead according to one of Claims 1 through 8, **characterized in that** a proximal electrode (12), which has a distance to the distal end (16) of the electrode lead body (10), is situated proximally to the distal electrode (20).

10. The electrode lead according to Claim 9, **characterized in that** the proximal electrode (12) is a ring electrode which encloses the lumen.

11. The electrode lead according to one of Claims 1 through 10 having a guide wire (14), **characterized in that** the guide wire has an external electrical insulation along the greatest part of its length and has an electrode surface on its distal end or in its proximity, which is electrically connected to a proximal end of the guide wire and is implemented as a stimulation and/or sensing electrode.

12. The electrode lead according to one of Claims 1 through 10, **characterized by** a second electrode lead, which is inserted into the lumen and is longitudinally displaceable in relation to the first electrode lead, having at least one ring or tip electrode on the distal end of the second electrode lead or in its proximity, the ring or tip electrode being implemented as a stimulation and/or sensing electrode and being electrically connected to a proximal end of the second electrode lead.

13. The electrode lead according to one of Claims 1 through 12, **characterized by** a seal (24) in the area of the distal end of the lumen, which is implemented to allow a guide wire or the like to be led through and to seal the lumen against penetration of blood.

14. The electrode lead according to Claim 13, **characterized in that** the seal is implemented as a lip seal (24).

## Revendications

1. Ligne d'électrode pour l'application intraluminaire ou intracardiaque pour le raccordement à un pacemaker implantable, un défibrillateur implantable ou un cardioverteur implantable, comprenant un corps de ligne d'électrode (10) étiré en longueur et souple en flexion, qui présente une extrémité proximale, sur laquelle est disposé un raccordement électrique pour l'établissement d'une liaison électrique avec un appareil d'électro-stimulation, et une extrémité distale (16), sur laquelle est disposée au moins une électrode distale (20 ; 20a, 20b), qui est reliée électriquement au raccordement électrique et présente une surface extérieure électroconductrice, et dans lequel est prévu un lumen pour la réception d'un fil de guidage (14) ou similaire, qui s'étend depuis une ouverture proximale dans la zone de l'extrémité proximale du corps de ligne d'électrode jusqu'à une ouverture distale (18) dans la zone de l'extrémité distale du corps de ligne d'électrode (10), l'ouverture distale (18) étant réalisée et disposée de telle sorte qu'un fil de guidage peut sortir par l'ouverture distale (10) du lumen, l'électrode distale (20 ; 20a, 20b) même ne présentant pas d'ouverture par laquelle le fil de guidage doit passer et l'électrode distale (20 ; 20a, 20b) et l'ouverture distale (18) étant disposées en décalage latéral par rapport à une direction longitudinale du corps de ligne d'électrode (10) au niveau de l'extrémité (16) distale du corps de ligne d'électrode (10) de telle sorte qu'un fil de guidage sortant par l'ouverture distale (18) est guidé en passant devant l'électrode distale (20 ; 20a, 20b) avec un décalage latéral, **caractérisé en ce que** l'ouverture distale (18) est disposée sans décalage longitudinal à côté d'une extrémité proximale de l'électrode distale ou avec un décalage longitudinal au niveau distal par rapport à l'extrémité proximale de l'électrode distale.

2. Ligne d'électrode selon la revendication 1, **caractérisée en ce que** l'électrode distale (20) est réalisée comme électrode Tip et est disposée sur l'extrémité distale du corps de ligne d'électrode.

3. Ligne d'électrode selon la revendication 2, **caractérisée en ce que** le corps de ligne d'électrode (19) est pré-courbé au moins au niveau de son extrémité distale de telle sorte que le corps de ligne d'électrode s'incurve au moins après l'enlèvement d'un fil de guidage au moins dans un plan défini par la direction longitudinale du corps de ligne d'électrode ainsi que l'ouverture distale et l'électrode distale en direction de l'électrode distale.

4. Ligne d'électrode selon la revendication 2, **caractérisée en ce que** deux électrodes distales (20a, 20b) sont disposées à côté de l'ouverture distale (18) sur l'extrémité distale du corps de ligne d'électrode.

5. Ligne d'électrode selon la revendication 4, **caractérisée en ce que** l'ouverture distale (18) est disposée de façon centrale entre les deux électrodes distales (20a, 20b).

6. Ligne d'électrode selon la revendication 2, **caractérisée en ce que** l'ouverture distale (18) est disposée avec un décalage dans la direction distale par rapport à une extrémité proximale de l'électrode distale.

7. Ligne d'électrode selon la revendication 6, **caractérisée en ce que** le corps de ligne d'électrode (10) présente dans une partie longitudinale proximale se trouvant à proximité de l'ouverture distale (18) un diamètre extérieur plus grand que l'électrode distale (20) dans sa partie longitudinale distale située au niveau distal de l'ouverture distale entre l'ouverture distale (18) et l'extrémité distale du corps de ligne d'électrode (10).

8. Ligne d'électrode selon la revendication 2, **caractérisée en ce que** l'ouverture distale (18) est disposée par rapport à la direction longitudinale du corps de ligne d'électrode (10) sans décalage longitudinal par rapport à l'extrémité proximale de l'électrode distale (20).

9. Ligne d'électrode selon l'une quelconque des revendications 1 à 8, **caractérisée en ce qu'**une électrode proximale (12), qui a une distance à l'extrémité distale (16) du corps de ligne d'électrode (10), est disposée à proximité de l'électrode distale (20).

10. Ligne d'électrode selon la revendication 9, **caractérisée en ce que** l'électrode proximale (12) est une électrode annulaire qui entoure le lumen.

11. Ligne d'électrode selon l'une quelconque des revendications 1 à 10, comprenant un fil de guidage (4), **caractérisée en ce que** le fil de guidage présente une isolation électrique extérieure le long de la plus grande partie de sa longueur et présente une surface d'électrode sur son extrémité distale ou à proximité de celle-ci, laquelle surface est reliée électriquement à une extrémité proximale du fil de guidage et est réalisée comme électrode de stimulation et/ou de palpation.

12. Ligne d'électrode selon l'une quelconque des revendications 1 à 10, **caractérisée par** une seconde ligne d'électrode introduite dans le lumen et coulissant dans la longueur par rapport à la première ligne d'électrode avec au moins une électrode annulaire ou une électrode à pointe sur l'extrémité distale de la seconde ligne d'électrode ou à proximité de son extrémité, l'électrode annulaire ou l'électrode à pointe étant réalisée comme électrode de stimulation et/ou comme électrode de palpation et étant reliée électriquement à une extrémité proximale de la seconde ligne d'électrode.

13. Ligne d'électrode selon l'une quelconque des revendications 1 à 12, **caractérisée par** un joint (24) dans la zone d'une extrémité distale du lumen, qui est réalisé pour permettre le passage d'un fil de guidage ou similaire et rendre étanche le lumen par rapport à la pénétration de sang.

14. Ligne d'électrode selon la revendication 13, **caractérisée en ce que** le joint est conçu comme joint à lèvres (24).
